(19) Europäisches Patentamt
European Patent Office
Office européen des brevets

(11) **EP 4 553 841 A1**

(12) **EUROPEAN PATENT APPLICATION**

(43) Date of publication:
**14.05.2025 Bulletin 2025/20**

(21) Application number: **23208454.1**

(22) Date of filing: **08.11.2023**

(51) International Patent Classification (IPC):
**G16H 15/00** (2018.01)   **G16H 30/40** (2018.01)
**G16H 40/67** (2018.01)

(52) Cooperative Patent Classification (CPC):
**G16H 30/40; G16H 15/00; G16H 40/67**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(71) Applicant: **Koninklijke Philips N.V.**
**5656 AG Eindhoven (NL)**

(72) Inventors:
• **WEESE, Rolf Jürgen**
**5656AG Eindhoven (NL)**

• **FLÄSCHNER, Nick**
**5656AG Eindhoven (NL)**
• **WENZEL, Fabian**
**5656AG Eindhoven (NL)**
• **EWALD, Arne**
**5656AG Eindhoven (NL)**
• **PETERS, Jochen**
**5656AG Eindhoven (NL)**

(74) Representative: **Philips Intellectual Property & Standards**
**High Tech Campus 52**
**5656 AG Eindhoven (NL)**

(54) **METHOD AND A DEVICE FOR GENERATING AT LEAST A PART OF A STRUCTURED MEDICAL REPORT**

(57) The present invention relates to method and a device for generating part of a structured medical report. The method comprises a stepwise creation and refinement of findings related to an examined body part of a subject based on keywords extracted from medical information, such as words spoken by a user.

FIG.4

EP 4 553 841 A1

**Description**

FIELD OF THE INVENTION

The present invention relates to a method and a device for generating at least a part of a structured medical report.

BACKGROUND OF THE INVENTION

Today, reporting is often done using voice recognition and free text, because this is a very efficient way. Structured reporting solutions are different since their content is built from a defined set of findings with properties that can have different options. They have the advantage of standardizing findings and their description which increases quality. In addition, standardized findings can easily be pre-populated using artificial intelligence (AI) methods that derive findings from the images of the examination. Nevertheless, structured reporting is often considered to be less efficient than free text reporting since the latter might be addressed by seamless integration of voice recognition.

SUMMARY OF THE INVENTION

It is an object of the present invention to provide an efficient way of generating at least a part of a structured medical report.

In a first aspect of the present invention a method for generating part of a structured medical report is presented, the method comprising the steps of:

a) obtaining medical information regarding an examined body part of the subject;
b) determining a finding related to the examined body part by extracting one or more keywords from the obtained medical information, wherein the keywords are predefined medical terms related to the examined body part;
c) defining a set of further keywords related to the examined body part based on the one or more extracted keywords and based on one or more predetermined rules, models and/or tables, the defined set of further keywords indicating further keywords that are allowed and/or further keywords that are prohibited for refinement of the finding;
d) obtaining further medical information regarding the examined body part of the subj ect;
e) refining the finding by extracting or specifying one or more further keywords from the defined set of further keywords and/or modifying one or more keywords of the finding based on the obtained further medical information;
f) iteratively performing steps c), d) and e) one or more times to further refine the finding; and
g) generating at least a part of a structured medical report of the examined body part based on the refined finding.

The refinement or specification of keywords in at least some of the embodiments refers to extraction of additional keywords from the set of further keywords, or they can be specified, such as determining a sub-set of keywords from the set of further keywords.

In further aspects of the present invention, there are provided a corresponding device, a computer program which comprises program code (or program code means) for causing a computer to perform the steps of the method disclosed herein when said computer program is carried out on a computer as well as a non-transitory computer-readable recording medium that stores therein a computer program product, which, when executed by a processor, causes the method disclosed herein to be performed.

Preferred embodiments of the invention are defined in the dependent claims. It shall be understood that the claimed device, computer program and medium have similar and/or identical preferred embodiments as the claimed method, in particular as defined in the dependent claims and as disclosed herein.

The present invention is based on the idea to provide an efficient way to enter new and/or modify previously generated structured findings using the extraction of keywords from medical information, for instance by use of voice recognition. An event (e.g., pressing a button, selecting an option by a computer mouse, or speaking a certain command) is used to start entering or modifying a finding including a sequence of properties with property-specific options. Findings are stepwise created and refined, e.g., based on words spoken by a user, which can be given without a predefined order. Voice recognition is used in an embodiment to recognize the keywords that correspond to an option of multiple keywords. These options of keywords are represented by a defined set of further keywords indicating further keywords that are allowed and/or further keywords that are prohibited for refinement of the finding. When creating or modifying of a finding is completed, the user may generate an event, for instance, to enter or modify another finding. It is to be understood that creating and determining can be used interchangeably within the present application. The structured findings are then used to generate a part of the medical report, particularly the part including the findings but possibly not parts including a diagnosis or impression.

In an embodiment the method further comprises the steps of obtaining image information of the examined body part of the subject and using the obtained image information in the step of creating and/or refining the finding. The use of the image information in addition to the keywords from the obtained medical information further improves the creation and/or refinement of findings. Hereby, the obtained image information may be used in the step of creating and/or refining the finding by checking if one or more further keywords extracted from the defined set of further keywords and/or one or more modified keywords fit with (e.g., correlates to) the obtained image information. The image information is generally medical image information, such as images of the body part obtained by any medical imaging modality (X-ray, CT, MRI, ultrasound, and others).

Preferably, the medical information is obtained in the form of textual and/or audio information, in particular written text and/or spoken words, and the keywords are extracted from the medical information by use of text recognition and/or speech recognition. This enables the user to speak freely and without any defined order. Text recognition may, e.g., be used to detect key words (e.g. via hash tables) in a sequence of words given as letters.

According to a further embodiment, the one or more keywords extracted from the medical information and/or included in the set of keywords relate to a hierarchical description of a part of the anatomy and/or a feature and/or abnormality. For instance, they relate to particular information on one or more of compartment, sub-compartment, type of structure, sub-structure, sub-sub-structure, pathology, grading, and one or more further properties of the examined body part of the subject. As an example, a hierarchical description of an anatomical part may be as follows: left knee (anatomical part) -> femoro-tibial (compartment) -> lateral part of femoro-tibial (sub-compartment) -> meniscus (in lateral part of femoro-tibial compartment; type of structure) -> pars intermedia of meniscus (sub-sub-structure).

In an embodiment, a trained algorithm, or a computing system, trained on extracting keywords from medical information for one or more (preferably for a plurality of) body parts are used for extracting the one or more keywords from the medical information and/or a trained algorithm or computing system trained on refining a finding is used for refining the finding. This may include the use of a trained speech or word recognition system and/or the use of a trained image analysis tool. For instance, a large amount of information (e.g., a large number of words and/or sentences spoken by a person) has been used for training an algorithm or computing system, and for each piece of information it has been indicated to the algorithm or computing system if it qualifies as medical information and/or if there any keyword(s) are included in the medical information. This represents one way of training the algorithm or computing system to recognize keywords and extract them from given information. Training of keyword recognition from voice is, e.g., described in Jean Louis K. E Fendji, Diane C. M. Tala, Blaise O. Yenke, Marcellin Atemkeng; Automatic Speech Recognition Using Limited Vocabulary: A Survey, 2022, 36(1), 2095039, which is herein incorporated by reference.

The trained algorithm or computing system may comprise a machine learning system, a neural network, a convolutional neural network, a classification network (e.g., a residual neural network) or a U-net-like network. For instance, previously defined options of the finding and findings derived via Neural Networks from the images of an examination may be considered. A property associated with an option may be identified and its option may be defined or overwritten. Such networks and systems are generally known in the art.

In another embodiment the keywords are extracted from the medical information by use of speech recognition using probabilities of keywords and/or combinations of keywords. For instance, the speech recognition (or voice recognition or word recognition) may provide one or more extracted keywords per keyword to be recognized. In an exemplary implementation, the recognition may result in an N-best list of potential keywords for a single keyword to be recognized, wherein N may, e.g., be in the range of N≥5 (e.g. up to 30). For the N-best list, probabilities / likelihoods for each element of the list should be given.

In another embodiment, information from image-based classifiers is combined for creating and/or refining a finding. This further improves the accuracy and reliability of the creation and refinement of findings. An image-based classifier may, e.g., segment the anatomy (e.g., pars intermedia of medial meniscus) and construct a region-of-interest around the segmentation result. The region of interest may be put into a classification network (e.g. ResNet (residual network)) and the classification result could, for instance, be the kind of damage (according to a medically accepted scale such as Stoller). For training, a set of images may be used where the damage of the pars intermedia of the medial meniscus according to Stoller is known, and ResNet coefficients may be optimized during training using, e.g., cross-entropy loss or any other commonly known loss function for classification.

In an implementation a probability formula

$$P(f|I) = 1 - \prod_{j \in p} \left(1 - P(f_j|I)\right)$$

is used for determining if the keyword fits with (e.g., correlates to) the finding in order to create and/or refine the finding. Optionally, it may be determined which keyword fits with the finding as well.

$f$ is a possibly incomplete finding (i.e., not all properties are defined); $f_j$ ($i$ in $p$) denotes all findings that are "compatible" with finding $f$; "compatible" means that the properties defined for $f$ are the same for $f_j$, but for $f_j$ also the

undefined properties are defined; $P(f_j|I)$ is the probability of finding $f_j$ given the image $I$ (e.g., computed with a classification network); $P(f|I)$ is the probability of the incomplete finding $f$ given image $I$ and computed from individual classifiers. For instance, when $f$ denotes a damage of the meniscus in the lateral part of the femoro-tibial compartment, and the image indicates that the probability of a damage in frontal horn, the lateral horn and the pars intermedia of this part of the meniscus is low (i.e. $P(f_j|I)\sim=0$), then also $P(f|I)$ is small. Some mechanism of this type may be used when the image-based classifiers are used in successively defining all the keywords.

Preferably, the at least a part of the structured medical report of the examined body part is generated by iteratively filling a report template and/or an existing medical report (e.g., a still incomplete medical report or a medical report that shall further be refined) based on iteratively refined findings. The template supports the generation of the medical report.

In an embodiment, visual and/or acoustical feedback is provided to a user presenting the finding and/or structured report.

The device according to the present invention for generating part of a structured medical report generally comprises circuitry configured to carry out the steps of the method according to the present invention. The circuitry may include one or more of a non-transitory computer readable medium, separate processing units, a computer, a mixture of hard- and software, a programmed processor, and similar ones.

In an implementation the device may further comprise one or more of:

- a user interface configured to receive user input and/or provide feedback and/or the structured medical report to the user;
- a medical information input configured to obtain the medical information regarding the examined body part of the subject;
- a rules input configured to obtain the one or more predetermined rules, models and/or tables;
- a recognition unit configured to extract one or more initial keywords from the medical information by use of text recognition and/or speech recognition; and
- a processing unit configured to generate the part of the structured medical report based on the obtained medical information, the obtained one or more predetermined rules, models and/or tables and the keywords.

The processing unit comprises one or more of:

- an image-based keyword extractor configured to extract one or more keywords from medical images;
- a keyword selector configured to select and/or modify one or more keywords based on keywords extracted from the medical images and/or recognized from text and/or speech of the user; and
- a findings generator configured to create and/or refine a finding based on keywords selected and/or modified by the keyword selector and the one or more predetermined rules, models and/or tables.

BRIEF DESCRIPTION OF THE DRAWINGS

These and other aspects of the invention will be apparent from and elucidated with reference to the embodiment(s) described hereinafter. In the following drawings:

Fig. 1 shows a schematic diagram of an embodiment of system according to the present invention;
Fig. 2 shows a schematic diagram of a first embodiment of device according to the present invention;
Fig. 3 shows a flow chart of an embodiment of method according to the present invention; and
Fig. 4 shows a schematic diagram of a second embodiment of device according to the present invention.

DETAILED DESCRIPTION OF EMBODIMENTS

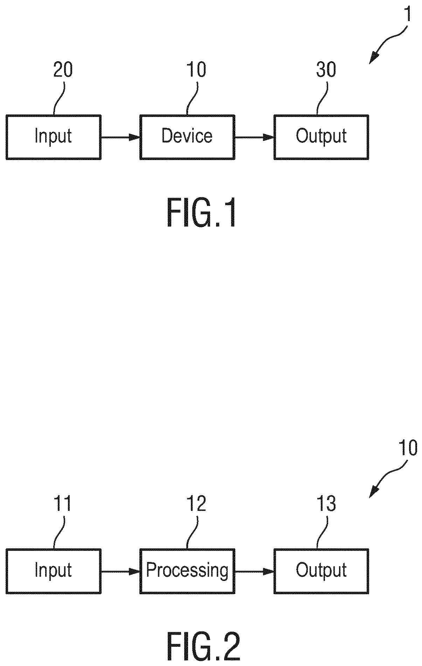

Fig. 1 shows a schematic diagram of an embodiment of a system 1 according to the present invention for generating part of a structured medical report for a subject who has undergone a medical examination. The system 1 may, e.g., be used by a medical practitioner (generally called "user"), such as a radiologist, who examines medical images (e.g. an MRI, CT or X-ray images) of a patient after a corresponding imaging scan of a certain body part (e.g. knee, brain, abdomen, etc.). The medical practitioner usually types or dictates (e.g. using speech recognition) a medical report, which is then stored along with the medical images in a database, e.g. a hospital's patient database. The system according to the present invention is configured to support the generation of at least a part of the medical report so that findings can be created more easily, and the medical practitioner can use free text.

The system 1 comprises a device 10 for generating part of a structured medical report, an input device 20 for providing medical information regarding an examined body part of the subject, and an output device 30 for outputting at least the generated part of the structured medical report.

The device 10 may be implemented by respective units or circuitry, e.g., a processor, processing circuitry, a computer, dedicated hardware, etc., that carries out the functions of the device. Alternatively, a common unit or circuitry, e.g., a common processor or computer, may implement the various functions of the device, or separate units or elements may be used that together represent the circuitry. In an exemplary implementation, a programmed processor may represent the device 10, which may execute a computer program that may be stored in a memory that is accessed by the processor.

The input device 20 is configured to provide medical information regarding the examined body part of the subject. It may be implemented by a microphone that is configured to record speech of the medical practitioner and/or other input means, such as a keyboard for typing information recorded by a computer. The speech and typed information thus represent the medical information.

The output device 30 is configured to output the generated part of the medical report. Generally, the output device 30 may generally be any means that outputs the generated medical report in visual (preferred) or audible form, e.g., in text form, as image or diagram, as sound or spoken words, etc. It may be implemented by a monitor that displays the medical report. Optionally, in addition, the obtained medical information and/or commands and/or information for the user may be outputted. In another implementation, the output may comprise an interface to a database or storage that stores the medical report. In general, the output device 30 may comprise a loudspeaker, a touchscreen, a computer monitor, the screen of a smartphone or tablet, etc.

Fig. 2 shows a schematic diagram of a first embodiment of device 10 for generating part of a structured medical report according to the present invention. In an exemplary implementation, a programmed processor or computer may represent the device 10, which may execute a computer program that may be stored in a memory that is accessed by the processor.

The device 10 comprises an input unit 11 configured to obtain the medical information. The input unit 11 may be directly coupled or connected to the input device 20 or may obtain (i.e., retrieve or receive) the medical information from a storage, buffer, network, or bus, etc. The input unit 11 may thus e.g. be a (wired or wireless) communication interface or data interface, such as a Bluetooth interface, WiFi interface, LAN interface, HDMI interface, direct cable connection, or any other suitable interface allowing signal transfer to the device.

The device 10 further comprises a processing unit 12 configured to generate the part of the medical report. The processing unit 12 may be any kind of means configured to process the signals and generate part of a medical report there from. It may be implemented in software and/or hardware, e.g., as a programmed processor or computer or app on a user device such as a smartphone, tablet, laptop, PC, workstation, and others.

The device 10 further comprises an output unit 13 configured to output the generated part of the medical report. The output unit 13 may generally be any interface that provides the medical report, e.g., transmits it to another device or provides it for retrieval by another device (e.g., a smartphone, computer, tablet, etc.). It may thus generally be any (wired or wireless) communication or data interface.

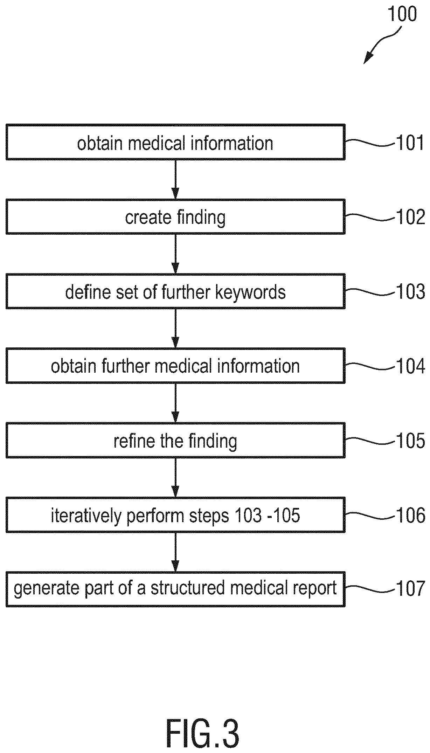

Fig. 3 shows a flow chart of a first embodiment of a method 100 for generating part of a structured medical report according to the present invention. The steps of the method 100 may be carried out by the device 10, wherein the main steps of the method are carried out by the processing unit 12. The method 100 may e.g. be implemented as computer program running on a computer or processor.

In a first step 101, medical information regarding an examined body part of the subject, for instance words spoken by the user with information about the examined body part. For instance, words spoken by a radiologist when reviewing a medical image e.g. of a patient's knee are received from a microphone used by the user. The spoken words are recognized e.g. by speech recognition and then further processed.

An event may signal the start of the whole method. For instance, a click on a button or an item on a screen by a mouse or a voice command like "start" may be given. Different possibilities to trigger the event (keyboard, mouse click, click on a screen item, voice command) may be available for user convenience. The same events can be used during the operation of the method for initiating certain steps or for stopping the operation or certain steps.

In a second step 102, a finding related to the examined body part is created by extracting one or more keywords from the obtained medical information, wherein the keywords are predefined medical terms related to the examined body part. Generally, keywords may include any terms (including acronyms and synonyms) that describe or are in any other way related to a body part and used by medical practitioners to describe any properties (such as size, texture, position, orientation, form, quality, and others) of a body part. Findings generally include a set of properties with property-specific options. The property-specific options can depend on the option defined for another property. Depending on the type of property, a property can be defined by a single option or by a set of feasible options. In addition, a property can be undefined. For illustration, Table 1 provides, as an example, an overview over meniscus and cartilage related findings in the knee. A complete set of findings for the knee covers many more findings, for instance, also findings related to bones and ligaments.

| | Compartment | sub-compartment | type of structure | sub-structure | subsub-structure(s) | pathology | grading | further properties |
|---|---|---|---|---|---|---|---|---|
| | | | | | Properties | | | |
| | ID | ID | ID | ID | Set of IDs | ID | ID | ID |
| Options | femoro-tibial | medial, lateral | meniscus | (not used) | anterior horn, pars intermedia, posterior horn | (not used) | Stoller 1, Stoller 2, Stoller 3, Stoller 4, Stoller 5 | only for Stoller 3: vertical, radial, horizontal, bucket-handle, flap, fold, other |
| | femoro-tibial | medial, lateral | meniscus | (not used) | (not used) | (not used) | Watanabe 1, Watanabe 2, Watanabe 3 | (not used) |
| | femoro-tibial | medial, lateral | meniscus | (not used) | (not used) | vacuole, other | (not used) | (not used) |
| | femoro-tibial | medial, lateral | cartilage | femoral | segment 1, segment 2,..., segment 5 | (not used) | ICRS/Valloton 1, ICRS/Valloton 2, ICRS/Valloton 3, ICRS/Valloton 4 | (not used) |
| | | | | tibial | segment 1, segment 2, segment 3 | | | |
| | femoro-patellar | (not used) | | (not used) | retropatellar, trochlear | | | |
| | femoro-tibial | medial, lateral | cartilage | femoral, tibial | (not used) | other | (not used) | (not used) |
| | femoro-patellar | (not used) | | (not used) | | | | |

<div align="center">

Table 1

</div>

To define a finding, in an embodiment the user stepwise fills in the properties of a finding via spoken keywords (that correspond to options) without a pre-defined order. In each step, a keyword $w^*$ refines or corrects a property of the current finding $f^{(k)}$. In addition, if an option for one property implicitly defines an option for another property, it is applied automatically. These steps are repeated until the finding is complete and correct.

In a third step 103, a set of further keywords related to the examined body part is defined based on the one or more extracted keywords and based on one or more predetermined rules, models and/or tables, the defined set of further keywords indicating further keywords that are allowed and/or further keywords that are prohibited for refinement of the finding. Table 2 illustrates the definition of a finding via keywords. The highlighted fields indicate the provided keywords. Terms in brackets in the highlighted fields indicate the options that are implied. The rows with the term "keywords" in the first column contain the available keywords for refining or correcting the finding. These rows are skipped for state 0 and 2. The rows with the expression "$f^{(..)}$" show refinement of a finding. The last row illustrates correction of a finding.

| State | Action | Compartment | sub-compartment | type of structure | sub-structure | subsub-structure(s) | pathology | grading | further properties |
|---|---|---|---|---|---|---|---|---|---|
| $f^{(0)}$ | refine | undefined | undefined | undefined | undefined | undefined | undefined | undefined | undefined |
| $f^{(1)}$ | refine | (femoro-tibial) | undefined | meniscus | (not used) | undefined | undefined | undefined | undefined |
| Keywords | | | medial, lateral | cartilage | | anterior horn, pars intermedia, posterior horn, (and all combinations) | vacuole, other | Stoller 1, Stoller 2, Stoller 3, Stoller 4, Stoller 5, Watanabe 1, Watanabe 2, Watanabe 3 | only for Stoller 3: vertical, radial, horizontal, bucket-handle, flap, fold, other |
| $f^{(2)}$ | refine | femoro-tibial | medial | meniscus | not used | Undefined | undefined | undefined | undefined |
| $f^{(3)}$ | refine | femoro-tibial | medial | meniscus | not used | pars intermedia & frontal horn | (not used) | undefined | undefined |
| Keywords | | | lateral | | | anterior horn, pars intermedia, posterior horn, (and all combinations) | | Stoller 1, Stoller 2, Stoller 3, Stoller 4, Stoller 5, | only for Stoller 3: vertical, radial, horizontal, bucket-handle, flap, fold, other |
| $f^{(4)}$ | refine | femoro-tibial | medial | meniscus | not used | pars intermedia & frontal horn | not used | (Stoller 3) | horizontal |
| Keywords | | | lateral | | | anterior horn, pars intermedia, posterior horn, (and all combinations) | | Stoller 1, Stoller 2, Stoller 3, Stoller 4, Stoller 5, | only for Stoller 3: vertical, radial, horizontal, bucket-handle, flap, fold, other |
| $f^{(5)}$ | correct | femoro-tibial | medial | meniscus | not used | pars intermedia & frontal horn | not used | Stoller 3 | bucket-handle |

Table 2

Generally, rules, models and/or tables may be used to define the further set of keywords after a finding has been generated or refined. Rules, models and/or tables may be predetermined, e.g., stored in a database that is, e.g., stored by the device 10 itself or on a server that is accessed by the device 10. They may be provided for multiple different body parts, such as anatomical objects, and may include all options that are generally used to describe any properties of the respective anatomical object after an examination. Rules, tables and models are different forms in which the set of further keywords that are available for further use and that may not be used, given the already given keywords and findings. For instance, a table may list, as shown above in Tables 1 and 2, combinations of keywords that are allowed to define a finding. Rules and a model may define, e.g., in the form of a computer program or flowchart, which next keyword may be used or may not be used based on keywords and/or findings that have been defined already.

In a fourth step 104, further medical information regarding the examined body part of the subject is obtained. For instance, the user speaks further words. In a fifth step 105, the finding is refined by extracting one or more further keywords from the defined set of further keywords (e.g., to describe further properties of the examined body part) and/or modifying one or more keywords of the finding (e.g., to correct a previous finding and/or keyword and/or to more precisely describe a property of the examined body part) based on the obtained further medical information. As shown in Table 2, there can be keywords allowed for properties of the finding that have already been defined. When such a keyword is recognized / identified, the finding is modified, and the keyword associated with the property is replaced by the most recent one.

Step 104 and/or step 105 may be initiated by an event as mentioned above. For instance, an event used to start modifying a finding might be by a click on a button / finding on the screen or a voice command "modify 3" to modify finding 3 etc. The event might also be a click on some anatomical structure in one of the images of the examination that opens a related finding or generates a new finding pre-populated with anatomical information (related information can e.g., derived by model-based segmentation from the image).

In a sixth step 106, steps 103-105 are iteratively performed one or more times to further refine the finding. The iteration may be stopped if the user does no longer provide any further medical information (e.g., the user stops speaking) or in case of an event as mentioned above (e.g., a given command or pressing a stop button) or if all fields of a template of a medical report are completely or sufficiently filled.

In a seventh step 107, at least a part of a structured medical report of the examined body part is generated based on the refined finding. Some other part of the medical report, like a diagnosis or therapy, is preferably generated in the conventional manner.

With reference to Table 2, the definition and refinement of a finding can be illustrated. In an initial state 0, the properties of the finding $f^{(0)}$ are undefined. As an example, a first keyword "meniscus" is provided. Table 1 shows that "meniscus" is related to the "femoro-tibial" "compartment", i.e., the property "compartment" is defined accordingly resulting in the finding $f^{(1)}$.

In state 1, the finding $f^{(1)}$ defines the set of keywords that are accepted in the next step when refining or correcting the finding. These keywords are defined by all options that are allowed given the properties defined so far. To enable corrections, options are added that would only modify the corresponding property without further side effects on other properties. For instance, for finding $f^{(1)}$ "cartilage" is added, but not "femoro-patellar", because "femoro-patellar" would also imply to change the "type of structure" which has been previously defined as "meniscus". The currently defined option of a property may also be added, meaning no change if the keyword for a currently defined option of a property is repeated. In general, the keywords that can be accepted for a given state of a finding can be defined based on a set of rules and the structure of findings (see Table 1) to enable an intuitive definition and modification of findings.

In state 2, the finding $f^{(2)}$ is obtained and the relevant anatomical part is refined to be the sub-structure "medial meniscus".

In state 3, the finding $f^{(3)}$ is obtained after defining the sub-sub-structures. In particular, "pars intermedia and frontal horn" have been provided as option. For the sub-sub-structures, all kinds of keyword combinations are accepted.

In state 4, the finding $f^{(4)}$ is completely defined. All possible keywords relate to the correction of the finding. Here, the allowed keywords include "Stoller1", ..., "Stoller 5" for correcting the grading, i.e., the grading can be refined, even if its property "horizontal" would be set to "not used" as a side effect.

In state 5, the finding $f^{(5)}$ is obtained after changing the "further property" "horizontal" to "bucket-handle".

While Table 2 indicates that a keyword might be available for refining a finding or not, also a statistical model with high probabilities for available keywords and low probabilities for keywords that are "not available" may be used.

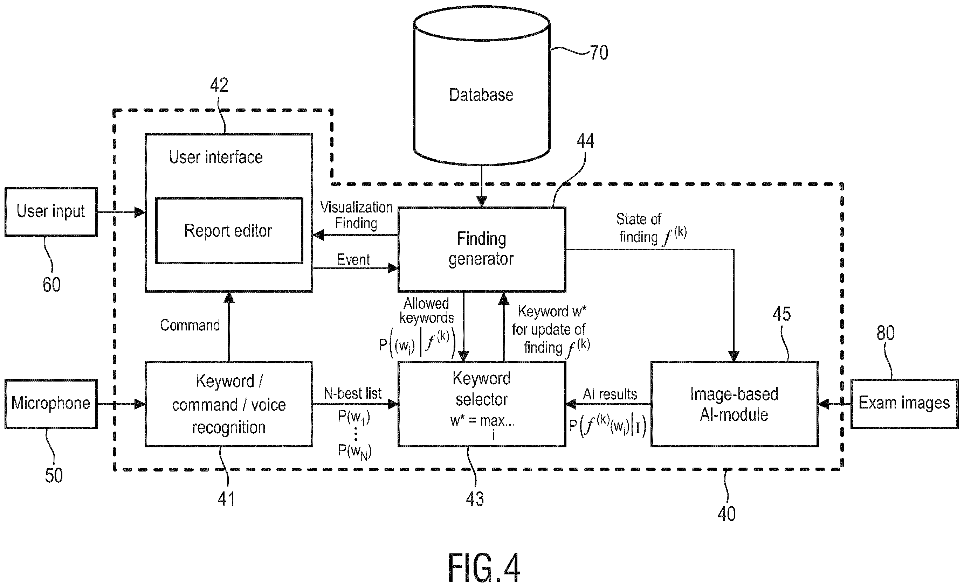

Fig. 4 shows a schematic diagram of a second embodiment of device 40 according to the present invention. From a microphone 50, spoken words are obtained and processed by the recognition unit 41 (including a medical information input that receives the spoken words from the microphone 50 or an intermediate buffer). In particular, keywords and commands (e.g. as events) are recognized. From a user input 60, e.g. a keyboard, computer mouse, touchscreen, etc., commands (events) may be received by a user interface 42, which receives any recognized events from the recognition unit 41 as well.

Based on one or more recognized keyword(s) an N-best list is generated by the recognition unit 41 indicating likelihoods (also called probability) that a recognized keyword corresponds to a certain keyword. In this example N likelihoods $P(w_1)$ to $P(w_N)$ are generated for N different options of keywords to which the recognized keyword corresponds.

A keyword selector 43 selects the keyword w* using e.g. a maximum-likelihood function, considering the N-best list and the allowed keywords for a particular finding. The selected keyword $w_*$ is provided to a finding generator 44 to create or update a finding $f^{(k)}$. The finding generator 44 may include a rules input that is configured to obtain the one or more predetermined rules, models and/or tables, e.g. by accessing a database 70 that stores a set of further keywords related to the examined body part and/or on one or more predetermined rules, models and/or tables The defined set of further keywords indicates further keywords that are allowed and/or further keywords that are prohibited for refinement of the finding. For instance, content of the type shown in Table 1 and Table 2 may be stored in the databased 70 and used by the finding generator 44.

The finding generator 44 feeds back allowed keywords to the keyword selector 43. The finding generator 44 may be triggered to start operation based on an event received from the user interface 42. The created or updated finding may be provided to the user interface 42 for visualization, e.g. on a screen (not shown).

In addition, an image-based module 45, e.g., an image-based AI module, may be provided that obtains one or more images of the examined body part of the subject, either directly from the imaging apparatus or from an image repository 80. Based on the one or more images, the keyword selection by the keyword selector 43 can be further improved. For instance, the obtained image information can be used to check if a keyword extracted from a defined set of further keywords fits with the obtained image information. For instance, if a keyword is included in the N-best list of keywords provided by the recognition unit but does not fit the image-based information provided by the image-based module 45, this keyword will not be selected by the keyword selector 43. The image-based module 45 may obtain the state of the finding from the finding generator 44 as well in order to select the image-based classifiers (i.e., the image-based module comprises a large number of classifiers, each indicating whether a structured finding is present or not) that must be evaluated and combine them into an overall probability / likelihood.

The voice-based definition of findings may follow the concept of the keyword-based definition of findings explained above. There is a current state of a finding $f^{(k)}$ and the next state $f^{(k+1)} = f^{(k)}(w^*)$ when the keyword or keyword combination w* is used. The state $f^{(k)}(w_i)$ is defined by defining or overwriting the corresponding property and adjusting all depending options to be consistent with the definition of the properties and options of findings (see Table 1 for an exemplary list). The conditional probability $P(w_i|f^{(k)})$ models the rules as discussed for the keyword-based definition of findings.

The overall probability of observing a sequence x of features extracted from the voice signal is modelled by

$$P\big(\underline{x}, w_i \big| f^{(k)}, I\big) = P\big(\underline{x}|w_i\big)P\big(w_i|f^{(k)}\big)\,P\big(f^{(k)}(w_i)|I\big)$$

where $P(\underline{x}|w_i)$ is the probability of observing a sequence $\underline{x}$ when the keyword or keyword combination $w_i$ is used. The probability $P(f^{(k)}(w_i)|I)$ describes the probabilities of AI-derived findings and biases recognition of keywords or keyword combination towards findings that can be found in the image for which findings should be defined.

Recognition of the keyword or keyword combination can be done by maximizing the probability

$$w^* = \max_i P\big(\underline{x}|w_i\big)P\big(w_i|f^{(k)}\big)\,P\big(f^{(k)}(w_i)|I\big)$$

using standard speech recognition methods for limited vocabulary as described, for instance, in Jean Louis K. E Fendji, Diane C. M. Tala, Blaise O. Yenke, Marcellin Atemkeng; Automatic Speech Recognition Using Limited Vocabulary: A Survey, 2022, 36(1), 2095039, which is hereby incorporated by reference. Alternatively, a speech recognition method may provide an N-best list $P(w_1), ..., P(w_1)$ as mentioned above, wherein the probabilities are re-weighted $P(w_i)P(w_i|f^{(k)})\,P(f^{(k)}(w_i)|I)$ and resorted resulting in a re-ordered N-best list, and the keyword or keyword combination with largest probability is selected. In case that no keyword exceeds a threshold for the re-weighted N-best list, a signal may be given that no proper word or phrase has been recognized.

The words and keyword combinations considered in the speech recognition method can be derived from the options in Table 2, which may be complemented with synonyms and phrases for defining a set of structures. For example, "Stoller 3" would be equivalent to "meniscus tear" (because the grading system Stoller is only used for meniscus).

In addition, specific voice commands (e.g., "next" to add another finding, "delete" to set a property to undefined to delete the finding that is currently entered (the idea how to modify a property of a finding is included in the logic of allowed keywords), ...) may be recognized. The probability $P(w_i|f^{(k)})$ is then defined as constant and not modulated with AI information, i.e., the command can be recognized for any state of a finding. In other words, next to keywords for defining the finding, there are also voice commands envisioned to indicate that a finding is complete (e.g. "done") and another one may be added (e.g. "next") or the finding should be deleted. The probability $P(w_i|f^{(k)})$ may be constant, but it may also be modulated. For instance, if a finding $f^{(k)}$ is incomplete, the probability $P(w_i|f^{(k)})$; $w_i$ = done or $w_i$ = next may be set to a low value. If image-based AI indicates that a finding is unlikely or the finding is already included in the report, the probability for the keyword "delete" may be set to a comparatively large value.

Probabilities may additionally be derived from AI-information. It is assumed that at least for a part of possible findings AI-algorithms $C(\{w_i|i \in f\}; I) \in [0,1]$ or, more generally, a trained algorithm or computing system are available that indicate presence of finding $f$ corresponding to properties $\{w_i|i \in f\}$. These AI-algorithms may, for instance, be hierarchically structured or classified by independent NN-based classifiers for individual findings with NN-response $C(\{w_i|i \in f\}; I) = P(f|I)$. In addition, in case a property with options $\{w_j|j \in p\}$ is undefined for finding $f$, the probability may be defined according to

$$P(f|I) = 1 - \prod_{j \in p}\Big(1 - P(f_j|I)\Big)$$

i.e., the probability of the finding is defined as the overall probability that none of the options applies. In case that no image-based AI algorithm is available for a finding, or a finding is only partially defined via AI (i.e., at least one property is undefined), $P(f|I)$ may be defined via statistics of findings over a population or using some assumptions of statistics of findings over a population. An exemplary trivial assumption may be that some findings have equal probability (e.g., population statistics for findings would only be available for groups of findings). In case a finding $f$ is already in the list of findings, its probability can be set to 0 to avoid that keyword or phrase recognition is biased towards a finding that has already been entered. $P(f|I)$ thus indicates the probability of a finding $f$ given image $I$ and may be computed via a neural network-based classifier.

Several types of visual and/or acoustical feedback may be provided when entering a finding. The status of the finding and (likely) options for keywords and phrases may be displayed. A visual and/or acoustical feedback might be provided when no word or phrase corresponding to the current state of the finding and the allowed voice commands can be recognized. Furthermore, a visual or acoustical signal can be given when a finding is completely defined. Generally, a finding is complete if, e.g., according to a scheme such as (but not limited to) Table 1, all properties of the finding are defined. Many other options of visual and/or acoustical feedback can be imagined.

In summary, according to the present invention an efficient way to define and modify structured findings using voice recognition is presented. An event can be used to start entering or modifying a finding consisting of a sequence of properties with property-specific options. The user stepwise fills in findings via spoken keywords that can be given without a pre-defined order. Voice recognition is used to recognize the keywords that correspond to an option. As additional key element, previously defined options of the finding and findings derived via Neural Networks from the images of the exam may be considered in this step. The property associated with an option is identified and its option is defined or overwritten. When entering or modifying of a finding is completed, the user can generate an event, for instance, to enter or modify another finding.

The benefit of the present invention is that users are able to rapidly complete a structured report with a sequence of keywords that are recognized with low error rate due to a restricted language set. By use of the presented device, system and method the user, e.g., a medical practitioner like a radiologist, is able to more efficiently, more comfortably and more quickly generate at least a part of a medical report. The predetermined rules, models and/or tables help a user to use appropriate keywords to stepwise create / modify findings and thus iteratively generate the desired part of the medical report.

Furthermore, limiting voice recognition to keywords and synonyms and the optional use of image derived classifiers reduces errors in voice recognition (due to dialect, misleading pronunciation, background noise, background conversations, and so on) and reduces time needed for corrections. It also makes dictation more comfortable as the user is understood better.

While the invention has been illustrated and described in detail in the drawings and foregoing description, such illustration and description are to be considered illustrative or exemplary and not restrictive; the invention is not limited to the disclosed embodiments. Other variations to the disclosed embodiments can be understood and effected by those skilled in the art in practicing the claimed invention, from a study of the drawings, the disclosure, and the appended claims.

In the claims, the word "comprising" does not exclude other elements or steps, and the indefinite article "a" or "an" does not exclude a plurality. A single element or other unit may fulfill the functions of several items recited in the claims. The mere fact that certain measures are recited in mutually different dependent claims does not indicate that a combination of these measures cannot be used to advantage.

A computer program may be stored/distributed on a suitable non-transitory medium, such as an optical storage medium or a solid-state medium supplied together with or as part of other hardware, but may also be distributed in other forms, such as via the Internet or other wired or wireless telecommunication systems.

Any reference signs in the claims should not be construed as limiting the scope.

It is to be understood that any of the previous steps described in relation to embodiments and/or training steps described above can be performed by a specific-purpose computer system or general-purpose computer system, or a computer-readable medium, or data carrier system configured to carry out any of the steps described previously. The computer system can include a set of software instructions that can be executed to cause the computer system to perform any of the methods or computer-based functions disclosed herein. The computer system may operate as a standalone device or may be connected, for example using a network, to other computer systems or peripheral devices. In embodiments, a computer system performs logical processing based on digital signals received via an analogue-to-digital converter.

Some portions of the description are presented in terms of symbolic representations of operations on non-transient signals stored within a computer memory. These descriptions and representations are used by those skilled in the data processing arts to convey the substance of their work most effectively to others skilled in the art. Such operations typically require physical manipulations of physical quantities. Usually, though not necessarily, these quantities take the form of electrical, magnetic, or optical signals capable of being stored, transferred, combined, compared, and otherwise manipulated. It is convenient at times, principally for reasons of common usage, to refer to these signals as bits, values, elements, symbols, characters, terms, numbers, or the like. Furthermore, it is also convenient at times to refer to certain arrangements of steps requiring physical manipulation of physical quantities as modules or code devices, without loss of generality.

The computer system further includes a main memory and a static memory, where memories in the computer system communicate with each other and the processor via a bus. Either or both main memory and the static memory may be considered representative examples of the memory of the controller, and store instructions used to implement some, or all aspects of methods and processes described herein. Memories described herein are tangible storage mediums for storing data and executable software instructions and are non-transitory during the time software instructions are stored therein. The main memory and the static memory are articles of manufacture and/or machine components. The main memory and the static memory are computer-readable mediums from which data and executable software instructions can be read by a computer (or e.g., the processor). Each of the main memory and the static memory may be implemented as one or more of random access memory (RAM), read only memory (ROM), flash memory, electrically programmable read only memory (EPROM), electrically erasable programmable read-only memory (EEPROM), registers, a hard disk, a removable disk, tape, compact disk read only memory (CD-ROM (Compact Disk - Read Only Memory)), digital versatile disk (DVD), floppy disk, Blu-ray disk, or any other form of storage medium known in the art. The memories may be volatile or non-volatile, secure and/or encrypted, unsecure and/or unencrypted.

"Memory" is an example of a computer-readable storage medium. Computer memory is any memory which is directly accessible to a processor. Examples of computer memory include RAM memory, registers, and register files. References to "computer memory" or "memory" should be interpreted as possibly being multiple memories. The memory may for instance be multiple memories within the same computer system. The memory may also be multiple memories distributed amongst multiple computer systems or computing devices. The memory may store various software applications including computer executable instructions, that when run on the processor, implement the methods and systems set out herein. Other forms of memory, such as a storage device and a mass storage device, may also be included and accessible by the processor (or processors) via the bus. The storage device and mass storage device can each contain any or all of the methods and systems discussed herein.

In accordance with various embodiments of the present disclosure, the methods described herein may be implemented using a hardware computer system that executes software programs. Further, in an exemplary, non-limited embodiment, implementations can include distributed processing, component/object distributed processing, and parallel processing. Virtual computer system processing may implement one or more of the methods or functionalities as described herein, and a processor described herein may be used to support a virtual processing environment.

The illustrations of the embodiments described herein are intended to provide a general understanding of the structure of the various embodiments. The illustrations are not intended to fully describe all the elements and features of the disclosure described herein. Many other embodiments may be apparent to those of skill in the art upon reviewing the disclosure. Other embodiments may be utilized and derived from the disclosure, such that structural and logical substitutions and changes may be made without departing from the scope of the disclosure. Additionally, the illustrations are merely representational and may not be drawn to scale. Certain proportions within the illustrations may be exaggerated, while other proportions may be minimized. Accordingly, the disclosure and the figures are to be regarded as illustrative rather than restrictive.

**Claims**

1. Method of generating at least a part of a structured medical report, the method comprising the steps of:

   a) obtaining medical information regarding an examined body part of the subject;
   b) determining a finding related to the examined body part by extracting one or more keywords from the obtained medical information, wherein the keywords are predefined medical terms related to the examined body part;
   c) defining a set of further keywords related to the examined body part based on the one or more extracted keywords and based on one or more predetermined rules, models and/or tables, the defined set of further keywords indicating further keywords that are allowed and/or further keywords that are prohibited for refinement of the finding;
   d) obtaining further medical information regarding the examined body part of the subj ect;
   e) refining the finding by specifying one or more additional keywords from the defined set of further keywords and/or modifying one or more keywords of the finding based on the obtained further medical information;
   f) iteratively performing steps c), d) and e) one or more times to further refine the finding; and
   g) generating at least a part of a structured medical report of the examined body part based on the refined finding.

2. Method according to claim 1,
   further comprising the step of obtaining image information of the examined body part of the subject; and
   using the obtained image information in the step of creating and/or refining the finding.

3. Method according to claim 2,
   wherein the obtained image information is used in the step of creating and/or refining the finding by checking if one or more further keywords extracted from the defined set of further keywords and/or one or more modified keywords fit with the obtained image information.

4. Method according to any one of the preceding claims,

   wherein the medical information is obtained in the form of textual and/or audio information, in particular written text and/or spoken words, and
   wherein the keywords are extracted from the medical information by use of text recognition and/or speech recognition.

5. Method according to any one of the preceding claims,
wherein the one or more keywords extracted from the medical information and/or included in the set of keywords relate to a hierarchical description of a part of the anatomy and/or a feature and/or abnormality, in particular information on one or more of the following: compartment, sub-compartment, type of structure, sub-structure, sub-sub-structure, pathology, grading, one or more further properties of the examined body part of the subject.

6. Method according to any one of the preceding claims,
wherein a trained algorithm, or a computing system, trained on extracting keywords from medical information for one or more body parts is used for extracting the one or more keywords from the medical information and/or a trained algorithm or computing system trained on refining a finding is used for refining the finding.

7. Method according to claim 6,
wherein the trained algorithm or computing system comprises a machine learning system, a neural network, a convolutional neural network, a classification network or a U-net-like network.

8. Method according to any one of the preceding claims,
wherein the keywords are extracted from the medical information by use of speech recognition, such as voice recognition, using probabilities of keywords and/or combinations of keywords.

9. Method according to any one of the preceding claims,
wherein information from image-based classifiers is combined for creating and/or refining a finding, and/or wherein a probability formula

$$P(f|I) = 1 - \prod_{j \in p} \left( 1 - P(f_j|I) \right)$$

is used for determining if the keyword fits with the finding in order to create and/or refine the finding.

10. Method according to any one of the preceding claims,
wherein the at least part of the structured medical report of the examined body part is generated by iteratively filling a report template and/or an existing medical report based on iteratively refined findings.

11. Method according to any one of the preceding claims,
wherein visual and/or acoustical feedback is provided to a user presenting the finding and/or structured report.

12. Computer program comprising program code for causing a computer to carry out the steps of the method as claimed in any one of preceding claims 1-11 when said computer program is carried out on the computer.

13. Device (10, 40) for generating at least a part of a structured medical report, the device comprising circuitry configured to:

a) obtain medical information regarding an examined body part of the subject;
b) create a finding related to the examined body part by extracting one or more keywords from the obtained medical information, wherein the keywords are predefined medical terms related to the examined body part;
c) define a set of further keywords related to the examined body part based on the one or more extracted keywords and based on one or more predetermined rules, models and/or tables, the defined set of further keywords indicating further keywords that are allowed and/or further keywords that are prohibited for refinement of the finding;
d) obtain further medical information regarding the examined body part of the subj ect;
e) refine the finding by extracting one or more further keywords from the defined set of further keywords and/or modifying one or more keywords of the finding based on the obtained further medical information;
f) iteratively perform steps c), d) and e) one or more times to further refine the finding; and
g) generate at least a part of a structured medical report of the examined body part based on the refined finding.

14. Device according to claim 13, wherein the circuitry comprises one or more of:

- a user interface (42) configured to receive user input and/or provide feedback and/or the structured medical report to the user;
- a medical information input (41) configured to obtain the medical information regarding the examined body part of the subject;
- a rules input (44) configured to obtain the one or more predetermined rules, models and/or tables;
- a recognition unit (41) configured to extract one or more initial keywords from the medical information by use of text recognition and/or speech recognition; and
- a processing unit (43, 44, 45) configured to generate the part of the structured medical report based on the obtained medical information, the obtained one or more predetermined rules, models and/or tables and the keywords.

15. Device according to claim 14, wherein the processing unit comprises one or more of:

- an image-based keyword extractor (45) configured to extract one or more keywords from medical images;
- a keyword selector (43) configured to select and/or modify one or more keywords based on keywords extracted from the medical images and/or recognized from text and/or speech of the user; and
- a findings generator (44) configured to create and/or refine a finding based on keywords selected and/or modified by the keyword selector and the one or more predetermined rules, models and/or tables.

20          10          30     1

| Input | → | Device | → | Output |

## FIG.1

11          12          13     10

| Input | → | Processing | → | Output |

## FIG.2

100

| obtain medical information | 101 |
| create finding | 102 |
| define set of further keywords | 103 |
| obtain further medical information | 104 |
| refine the finding | 105 |
| iteratively perform steps 103 -105 | 106 |
| generate part of a structured medical report | 107 |

FIG.3

FIG.4

## EUROPEAN SEARCH REPORT

Application Number

**EP 23 20 8454**

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2019/139642 A1 (ROBERGE JAMES [US] ET AL) 9 May 2019 (2019-05-09) * paragraphs 15, 52, 25, 105- 110, 136, 137, 115, 116, 114, 59, 97 figures 1-6 * | 1-15 | INV. G16H15/00 G16H30/40 G16H40/67 |
| X | WO 2023/279199 A1 (A I VALI INC [CA]) 12 January 2023 (2023-01-12) * paragraphs 158-190 figures 4, 5A claim 11 * | 1-15 | |
| A | JEAN LOUIS K. E FENDJI: "Automatic Speech Recognition Using Limited Vocabulary: A Survey", APPLIED ARTIFICIAL INTELLIGENCE, vol. 36, no. 1, 25 July 2022 (2022-07-25), XP093156007, US ISSN: 0883-9514, DOI: 10.1080/08839514.2022.2095039 * section "Language model" * | 1-15 | TECHNICAL FIELDS SEARCHED (IPC) G16H |

The present search report has been drawn up for all claims

| Place of search | Date of completion of the search | Examiner |
|---|---|---|
| Munich | 29 April 2024 | Wittke, Claudia |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 23 20 8454

This annex lists the patent family members relating to the patent documents cited in the above-mentioned European search report.
The members are as contained in the European Patent Office EDP file on
The European Patent Office is in no way liable for these particulars which are merely given for the purpose of information.

29-04-2024

| Patent document cited in search report | | Publication date | Patent family member(s) | | Publication date |
|---|---|---|---|---|---|
| US 2019139642 | A1 | 09-05-2019 | EP | 3448232 A1 | 06-03-2019 |
| | | | US | 2019139642 A1 | 09-05-2019 |
| | | | WO | 2017189758 A1 | 02-11-2017 |
| WO 2023279199 | A1 | 12-01-2023 | CA | 3223508 A1 | 12-01-2023 |
| | | | CN | 117836870 A | 05-04-2024 |
| | | | EP | 4367683 A1 | 15-05-2024 |
| | | | WO | 2023279199 A1 | 12-01-2023 |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **JEAN LOUIS K. E FENDJI** ; **DIANE C. M. TALA** ; **BLAISE O. YENKE** ; **MARCELLIN ATEMKENG**. *Automatic Speech Recognition Using Limited Vocabulary: A Survey*, 2022, vol. 36 (1), 2095039